# EUROPEAN PATENT SPECIFICATION

(11) **EP 2 059 295 B1**
(45) Date of publication and mention of the grant of the patent: **01.05.2013**
(21) Application number: 07710263.0
(22) Date of filing: 23.01.2007
(51) Int. Cl.: A61N 1/05

(54) **NOVEL ASSEMBLY METHODS FOR MEDICAL ELECTRICAL LEADS**
NEUE MONTAGEMETHODEN FÜR MEDIZINISCHE ELEKTRISCHE LEITUNGEN
NOUVEAUX PROCÉDÉS D'ASSEMBLAGE POUR DES FILS CONDUCTEURS ÉLECTRIQUES MÉDICAUX

(30) Priority: 21.08.2006 US 465941; 21.08.2006 US 465946; 21.08.2006 US 465879
(43) Date of publication of application: 20.05.2009
(73) Proprietor: Medtronic, Inc., Minneapolis, MN 55432-5604 (US)
(72) Inventor: SKUBITZ, Sean, P., Forest Lake, MN 55025 (US); BOATWRIGHT, Mary, L., Andover, MN 55304 (US); BOLEA, Stephen, L., Watertown, MN 55388 (US); TOWER, Jessica, L., Minneapolis, MN 55406 (US); METZLER, Michael, E., Minneapolis , MN 55408 (US)
(74) Representative: Hughes, Andrea Michelle
(86) International application number: PCT/US2007/060886
(87) International publication number: WO 2008/024524

(56) References cited:
- WO-A-99/56817
- WO-A-02/072192
- US-A- 3 738 368
- US-A1- 2005 090 885

## Description

### TECHNICAL FIELD

The present invention is related to medical electrical leads and more particularly to methods for making electrode assemblies thereof.

### BACKGROUND

Medical electrical leads include one or more conductors that extend within an elongate insulative body and are coupled to one or more electrodes supported by the body. The one or more electrodes are typically mounted to a distal portion of the lead body and the distal portion positioned, or implanted, in a patient's body to provide electrical stimulation, for example, within a pericardial space, to provide restorative cardiac stimulation, or, within an epidural space, to provide pain-relieving spinal stimulation.

The portion of the lead body that supports the one or more electrodes should be configured to, at minimum, allow each electrode surface to make contact with a target stimulation site, support each joint between the one or more electrodes and the corresponding conductor, and, in the case of more than one electrode, electrically isolate the electrodes and conductors from one another. Electrode assemblies have been developed, for example, within the context of the exemplary stimulation scenarios referenced above, wherein a 'flattened', or relatively thin, lead body portion, for example, having a patch or paddle configuration, supports one or more electrodes, preferably an array of electrodes, that are disposed along a major surface of the lead body portion. Document WO 02/072192 discloses a method for making a medical electrical lead according to the preamble of claim 1. However there is still a need for assembly methods that result in configurations, which meet the above criteria, and improve operational efficiency in manufacturing. This object is achieved with a method for making an medical electrical lead assembly according to claim 1.

### BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.

Figure 1A is a plan view of an exemplary medical electrical lead, according to some embodiments of the present invention.

Figure 1B is an end view of the lead shown in Figure 1A.

Figure 2A is a perspective view of a conductive component, according to some embodiments of the present invention.

Figure 2B is a plan view of a conductive component, according to some ' embodiments of the present invention.

Figure 3A-B are section views taken through section line D-D of Figure 1A, according to some alternate embodiments of the present invention.

Figure 3C is a perspective view of a conductive component, according to an alternate embodiment of the present invention.

Figure 4A is a perspective view of an insulative carrier showing a first side thereof, according to some embodiments of the present invention.

Figure 4B is a perspective view of an insulative carrier showing a second side thereof, according to some embodiments of the present invention.

Figure 4C is an enlarged view of a portion of the carrier shown in Figure 4B.

Figure 4D is an end view of the carrier shown in Figures 4A-C.

Figure 5A is a plan view of a portion of an electrode assembly, according to some embodiments of the present invention.

Figure 5B is a plan view of a portion of an electrode assembly, according to some alternate embodiments of the present invention.

Figure 5C is a plan view of a portion of an electrode assembly, according to yet further alternate embodiments.

Figures 6A-B are perspective views of a conductive component, according to further alternate embodiments of the present invention.

Figures 7A-B are section views taken through section line D-D of Figure 1A, according to further alternate embodiments of the present invention.

Figure 8 is a flow chart outlining methods of the present invention.

### DETAILED DESCRIPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes are provided for selected elements, and all other elements employ that which is known to those of skill in the field of the invention. Those skilled in the art will recognize that many of the examples provided have suitable alternatives that can be utilized.

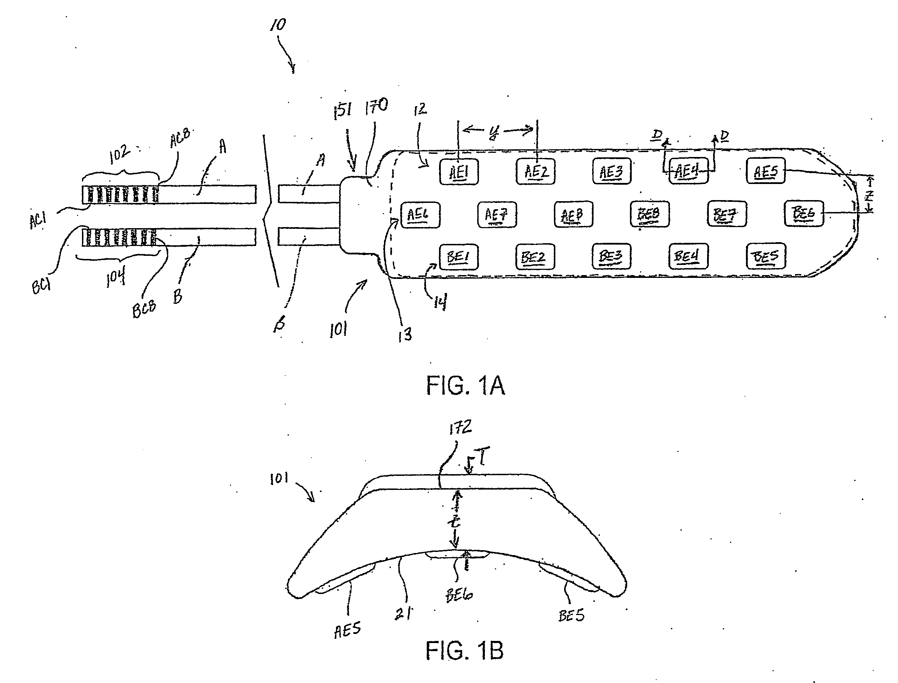

Figure 1A is a plan view of an exemplary medical electrical lead 10, according to some embodiments of the present invention; and Figure 1B is an end view of lead 10. Figure 1A illustrates lead 10 including a pair of elongate insulative tubular bodies A and B which are terminated at a proximal end by connectors 102 and 104, respectively, and which extend distally to terminate in an insulative paddle-shaped body 101 supporting an array of electrodes AE1-8 and BE1-8, which are arranged in three columns 12, 13, and 14. Figure 1B illustrates a profile of body 101, which has a maximum thickness T of approximately 0.09 inch (2.286 mm) along a proximal portion 151 thereof, and a maximum thickness t of approximately 0.075 inch (1.905 mm) along a length thereof that extends distally from proximal portion and corresponds with an extent of columns 12, 13, 14. The shape and profile of body 101 makes lead 10 suitable for epidural implantation to provide spinal cord stimulation; and the arrangement of electrodes AE1-8 and BE1-8 can provide flexibility for selection of a stimulation pattern from a variety of stimulation patterns after lead 10 is implanted, without having to physically reposition lead 10. According to an exemplary embodiment of the present invention, each electrode in columns 12, 13 and 14 are spaced apart from one another, along a length of each column, by a distance x, center-to-center, which is approximately 0.120 inch (3.048 mm), and each column 12, 13, 14 is spaced apart from one another by a distance z, center-to-center, which is approximately 0.179 inch (4.5466 mm). Although the electrode array illustrated herein provides a suitable example for preferred embodiments of the present invention, it should be noted that various alternate embodiments of the present invention include any number of electrodes in any arrangement.

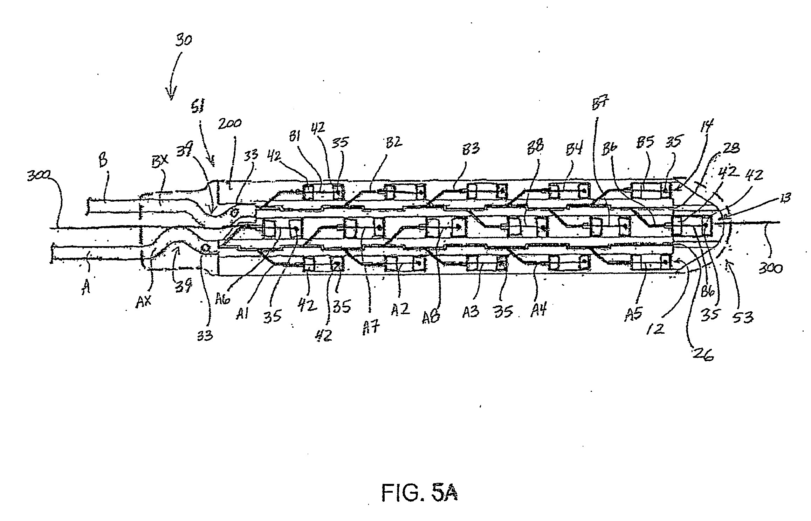

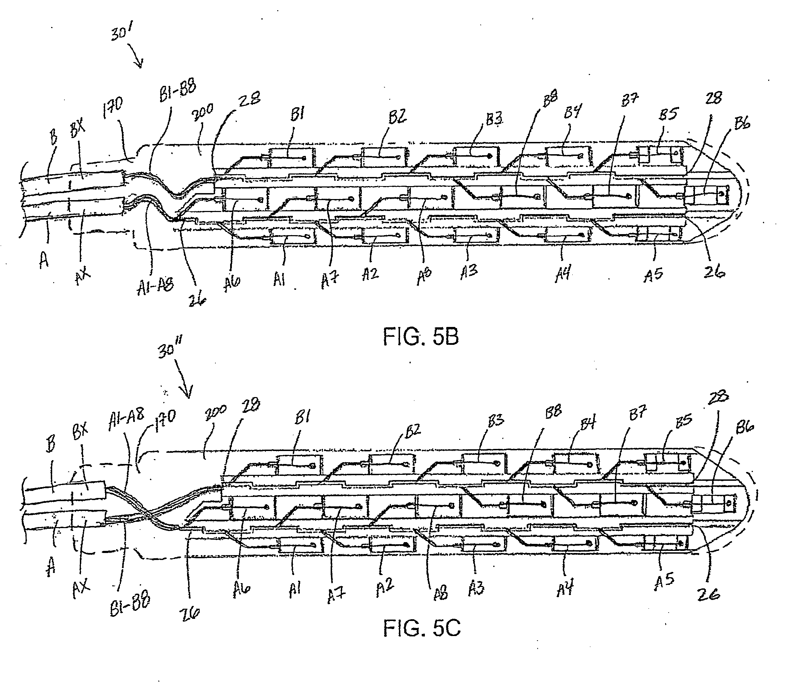

According to the illustrated embodiment, electrodes AE1-8 are coupled to corresponding contacts AC1-8 of connector 102 and electrodes BE1-8 are coupled to corresponding contacts BC1-8 of connector 104, such that each electrode may be independently powered when the connectors 102, 104 are plugged into a stimulation device. Although not shown, those skilled in the art will appreciate that conductors A1-8 (Figure 5), which couple each of electrodes AE1-8 to a corresponding contact of connector 102, extend within one or more longitudinally extending pre-formed channels, or lumens, of tubular body A, and conductors B1-8 (Figure 5), which couple each of electrodes BE1-8 to a corresponding contact of connector 104, extend within a similar one or more channels, or lumens, of tubular body B. Each of conductors A1-8 and B1-8 may be in the form of a coil or cable, being formed, for example from MP35N alloy, and each preferably includes an insulating jacket extending thereover, being formed, for example, from a fluoropolymer; such conductors are well known to those skilled in the art of medical electrical leads. A routing of the conductors within body 101, according to one embodiment of the present invention, will be described below, in conjunction with Figure 5.

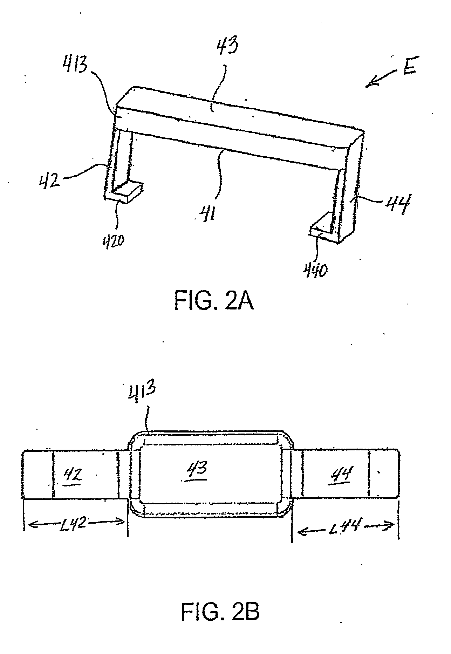

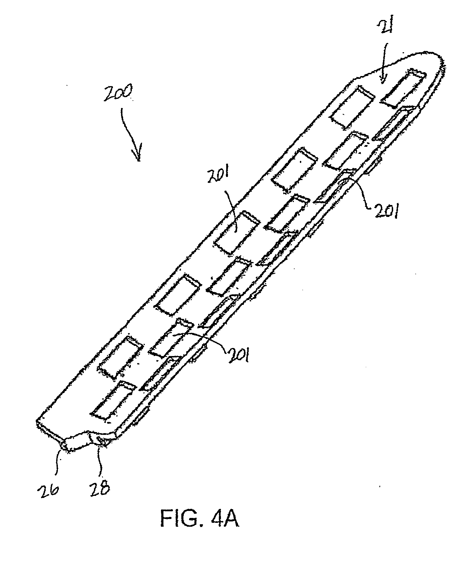

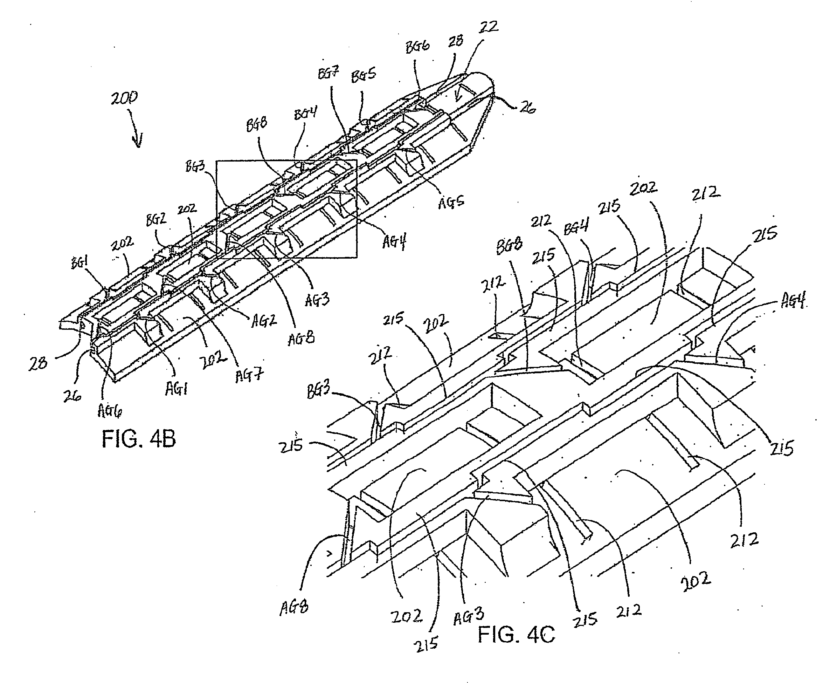

Figure 1A further illustrates, with a dashed line, a border of an insulative carrier 200 (Figures 4A-D), preferably formed from a flexible polymer, for example, silicone rubber which may have a polyester mesh panel embedded therein. According to the illustrated embodiment, carrier 200 is coupled to an insulative layer 170 to form body 101, and layer 170 is preferably formed from a material similar to that which forms carrier 200. Figure 1B further illustrates a first side 21 of carrier 200 corresponding to a side of body 101 from which electrodes AE1-8, BE1-8 may protrude, as illustrated; alternately, electrodes AE1-8, BE1-8 may be flush with first side 21 or recessed within first side 21. Insulative layer 170 is shown extending over a second side 22 (Figure 4B) of carrier 200 to form an opposite side 172 of body 101, and further extends about distal portions of bodies A,B to form proximal portion 151, for example, as described below in conjunction with Figure 5. According to embodiments of the present invention, each electrode AE1-8, BE1-8 is a portion 413 of a conductive component E, preferably formed from a 90/10 Platinum/iridium alloy, various embodiments of which are described in conjunction with Figures 2A-B, 3A-C, 6A-B and 7A-B.

Figures 2A-B illustrate electrode portion 413 of component E including an outward facing contact surface 43 and an inward facing surface 41; a pair of tabs 42, 44 extend from electrode portion 413 and are adapted to extend through carrier 200 in order to couple component E thereto. Figure 2A further illustrates each tab 42, 44 including a projection 420, 440, respectively. According to some embodiments of the present invention, component E is formed, for example, by stamping, such that portion 413, tabs 42, 44 and projections 420, 440 are approximately co-planar with one another, for example as shown in Figure 2B; then, tabs 42, 44 and projections 420, 440 are folded or bent away from portion 413 into the configuration illustrated in Figure 2A. Creases or indentations, for example, as illustrated by dashed lines in Figure 2B, may be formed in tabs 42, 44 in order to guide subsequent folding or bending. Alternately, component E may be provided as illustrated in Figure 2A by a single forming step, for example, machining or any other suitable forming method known to those skilled in the art. According to exemplary embodiments of the present invention, electrode portion 413 has a length, from tab 42 to tab 44, of approximately 0.185 inch (4.699 mm), and a width of approximately 0.086 inch (2.1844 mm); and tabs 42, 44 each have a thickness of approximately 0.005 inch (0.127 mm), and a length, from electrode portion 413 to projections 420, 440, respectively, of approximately 0.094 inch (2.3876 mm); and projections each have a length of approximately 0.028 inch (0.7112 mm).

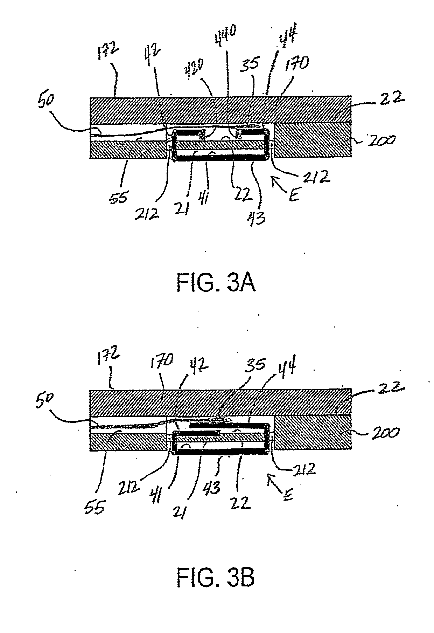

Figure 3A is a section view, through section line D-D of Figure 1A, wherein component E is coupled to carrier 200, according to some embodiments. Figure 3A illustrates each of tabs 42, 44, having been inserted through openings 212 of carrier 200, extending to second side 22 of carrier 200 where tabs 42, 44 are bent toward a surface of second side 22, such that tabs 42, 44 extend along the surface of second side 22, opposite inward facing surface 41 of the electrode portion that is disposed against a surface of first side 21 of carrier 200, and projections 420, 440 extend toward the surface of side 22. Pre-formed openings, for example, openings 212, may be sized to accommodate tabs 42, 44 including projections 420, 440, or any other tab cross-section for that matter; or, carrier 200 may be stretched to widen the pre-formed openings for insertion of tabs 42, 44 including projections 420, 440, or tabs having larger cross-sections that the openings. According to alternate embodiments, tabs 42, 44 do not include projections 220, 240. Furthermore, it should be noted that embodiments of the present invention need not include pre-formed openings, for example, openings 212 in carrier 200 for tabs 42, 44 to pass through, since tabs 42, 44 may form the openings by piercing carrier 200 upon insertion therethrough.

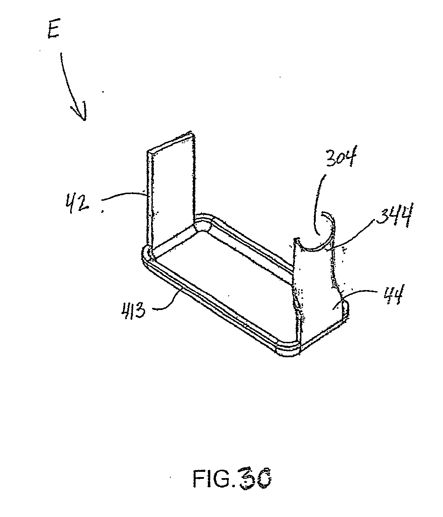

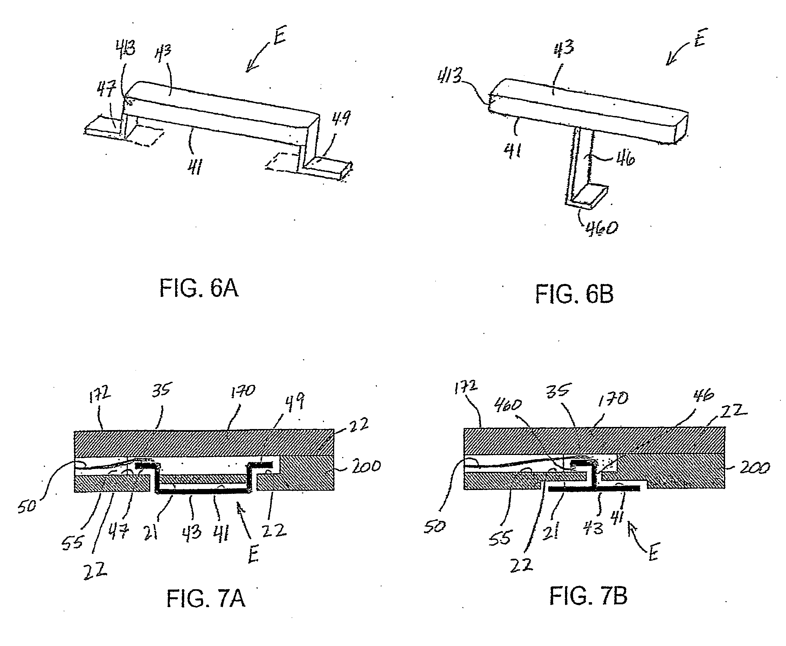

Referring back to Figure 2B, a length L42, L44 of each tab 42, 44, respectively, is indicated. According to further alternate embodiments of the present invention, length L44 is greater than length L42 such that when tabs 42, 44 are bent toward the surface of second side 22, tab 44 overlaps tab 42, as illustrated in Figure 3B. According to an exemplary embodiment wherein tabs overlap, a length of electrode portion 413, between tabs 42, 44 is approximately 0.185 inch (4.699 mm), length L44 of tab 44 is approximately 0.195 inch (4.953 mm), and length L42 of tab 42 is approximately 0.167 inch (4.2418). (Another alternate embodiment of component E is described below, in conjunction with Figure 3C. Further embodiments of component E coupled to carrier 200, which generally correspond to additional alternate embodiments of component E, shown in Figures 6A-B, are shown in Figures 7A-B, and are also described below.)

According to the embodiments illustrated in Figures 3A-B, the surface of the first side 21, against which surface 41 of electrode portion 413 is disposed, is recessed; and, with reference to Figure 4A, it may be seen that first side 21 of carrier 200 includes a recess 201 for each electrode portion 413, for example electrodes AE1-8 and BE1-8 (Figure 1A). It should be noted that embodiments of the present invention need not include recesses 201. Figures 3A-B further illustrate a conductor 50 coupled to tab 44 of component E by a joint 35, which may be any suitable type of joint known to those skilled in the art, for example, a weld or a crimp or a combination thereof wherein a sleeve is crimped to conductor 50 and then the crimped sleeve is welded to tab 44. Although Figures 3A-B show joints 35 formed on a surface of tab 44 that faces away from second side 22, some alternate embodiments include joints formed along a surface of a tab that faces toward second side 22 of carrier 200, when the tab is bent to couple the corresponding component E to carrier 200. According to one alternate embodiment of component E, tab 44 is pre-formed to accommodate crimping of conductor 50 thereto, for example as illustrated in Figure 3C. Figure 3C shows tab 44 including a curved portion 344 forming a groove 304, into which groove 304 conductor 50 may be inserted for crimping therein, either prior to, or following, the bending of tab 44 to couple component E to carrier 200. It should be noted that, although tab 42 is shown, in Figure 3C, without a similar a pre-formed curve, tab 42 may also be pre-formed as tab 44.

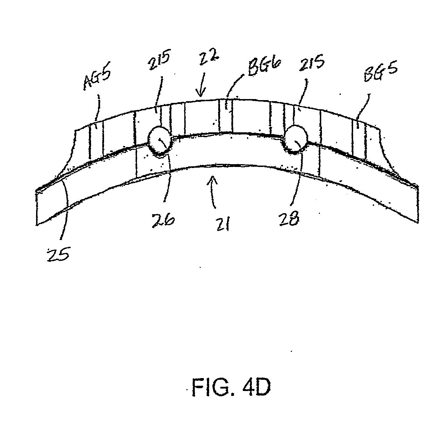

Referring back to Figures 3A-B, conductor 50 is shown extending from joint 35 into a pre-formed channel 55 along second side 22 of carrier. With reference to Figure 4B, an entirety of second side 22 of carrier 200, according to one embodiment, may be seen. Figure 4B illustrates a plurality of pre-formed channels AG1-8 and BG1-8 (each corresponding to channel 55 illustrated in Figures 3A-B and 7A-B); each channel AG1-8 and BG1-8 is shown extending from a corresponding pre-formed recess 202 that accommodates a pair of tabs 42, 44. According to the illustrated embodiment, channels AG1-8 and BG1-8 direct each conductor 50 from the corresponding joint 35 (Figures 3A and 5) into either a first or second longitudinally extending pre-formed channel 26, 28. Figure 4B shows first longitudinally extending pre-formed channel 26 extending from each of channels AG1-8, and second longitudinally extending pre-formed channel 28 extending from each of channels BG 1-8. Figure 4C is an enlarged view of a portion of carrier 200 enclosed in the box shown in Figure 4B. With reference to Figure 4B-C, it may be seen that a series of flaps 215 extend in a zipper-like fashion over each of channels 26 and 28; conductors 50 may be pressed across flaps 215 into channels 26,28 and then held in place by flaps 215. Figure 4D is a distal end view of carrier 200 showing flaps 215 extending over channels 26 and 28.

Figure 4D further illustrates a panel 25, for example, formed from a polyester mesh material, extending just beneath recessed surfaces of second side 22 of carrier 200. According to some alternate embodiments, panel 25 extends just beneath an exterior surface of first side 21. According to preferred embodiments of the present invention, panel 25 is integrally formed with carrier 200, having a foot print similar to that of carrier 200, for example, as defined by the dashed lines in Figure 1A, to provide some additional tear resistance to carrier 200.

Figure 5A is a plan view of an electrode assembly 30, incorporating carrier 200, wherein conductors 50 (Figures 3A-B and 7A-B) are designated as a first plurality A1-8 and a second plurality B1-8, and dashed lines indicate a border of insulative layer 170, which has been made transparent in order to show a routing of conductors A1-8 and B1-8. Figure 5A illustrates both of channels 26 and 28 extending approximately parallel to a longitudinal axis 300 of carrier 200, channel 26 between columns 12 and 13 to direct conductors A1-8 into tubular body A, and channel 28 between columns 13 and 14 to direct conductors B1-8 into tubular body B. According to the illustrated embodiment, each joint 35 is provided with some strain-relief against longitudinal loading by pre-formed channels AG1-8 and BG1-8 (Figure 4B), which direct conductors A1-8 and B1-8, at an angle to longitudinal axis 300, away from respective joints 35, and into respective longitudinal channels 26, 28.

Figure 5A further illustrates tubular bodies A and B each including a distal portion AX and BX, respectively; and, with reference to Figure 5A in conjunction with Figure 1A, it may be appreciated that portions AX and BX extend into proximal portion 151 of body 101 formed by insulative layer 170; layer 170 may further extend into pre-formed apertures 33 formed in sidewalls of portions AX, BX. It should be understood that portions AX and BX form extensions of the one or more longitudinal channels, or lumens, of tubular bodies A, B so that conductors A1-8 and B1-8, respectively may pass proximally therethrough from channels 26, 28. Figure 5A further illustrates each portion AX, BX including a curved segment 39, wherein segments 39 are longitudinally offset from one another, and an apex of each segment 39 is approximately aligned with one another in close proximity to longitudinal axis 300. The illustrated u-shape of each segment 39, and the arrangement of segments 39 may facilitate compact coupling of segments 39 to carrier 200, within layer 170; however, the scope of the present invention is not so limited, and any suitable curved shape and arrangement can be incorporated in alternate embodiments. Curved segments 39 may provide addition strain-relief and minimize flex-fatigue for the conductors A1-8, B1-8, and may further prevent longitudinal forces from dislodging electrode assembly 30 from an implanted location. According to exemplary embodiments, tubular bodies A, B are each formed from a polyurethane material and insulative layer 170 is formed from a silicone material; because silicone and polyurethane materials may not bond to one another, the mechanical interlocking between layer 170 and tubular bodies A, B, at apertures 33 and along curve segments 39, may provide additional structural integrity to assembly 30, for these exemplary embodiments.

Figures 5B-C are plan views of alternative electrode assemblies 30' and 30", respectively. Figures 5B-C illustrate portions AX and BX, of tubular bodies A and B, respectively, within insulative layer 170, including no curved segments, such as segments 39 illustrated in Figure 5A. According to the embodiment of Figure 5B, first plurality of conductors A1-8 are formed in a bend between channel 26 and portion AX, and second plurality of conductors B 1-8 are likewise formed in a bend between channel 28 and portion BX. According to the embodiment of Figure 5C, first plurality of conductors A1-8 cross over from channel 26 to enter tubular body B and second plurality of conductors B1-8 cross over from channel 28 to enter tubular body A. Conductors A1-8 and B 1-8 may be held in the illustrated configurations by additional channels formed in carrier 200 or may be positioned as such prior to molding of insulative layer 170 thereover. It should be noted that embodiments of the present invention may further include those in which conductors A1-8, B1-8 extend in approximately straight paths from channels 26 and 28 into respective tubular bodies A, B. It should be noted that each conductor of pluralities A1-8 and B 1-8 are electrically isolated from one another by an insulating jacket extending around each conductor.

Although Figures 5A-C illustrates a preferred embodiment wherein joints 35 are made between a distally located tab of each component E and the corresponding conductor, the scope of the present invention is not limited to any particular orientation of tabs 42 and 44, with respect to proximal and distal ends 51 and 53, respectively, of carrier 200; for example, some alternate embodiments include conductive components E oriented in carrier such that tabs 42, 44 are disposed on opposite sides of a longitudinal axis of the corresponding column 12, 13, 14. According to further alternate embodiments, joints 35 are made with the proximally located tabs shown in Figure 5A; or joint 35 may be made with a centrally located tab, for example, tab 46 shown in Figure 7B for the embodiment of component E illustrated in Figure 6B.

Figures 6A-B are perspective views of conductive component E, according to additional alternate embodiments of the present invention; and Figures 7A-B are section views taken through section line D-D of Figure 1A, according to embodiments that generally correspond to the embodiments of components E illustrated in Figures 6A-B, respectively. Figure 6A illustrates component E including tabs 47 and 49 extending outward such that tabs 47, 49, when component E is coupled to carrier 200, extend along a surface of second side 22 of carrier 200, which is offset from inward facing surface 41 of electrode portion 413, for example as illustrated in Figure 7A. According to the illustrated embodiment, tabs 47, 49 are pre-formed prior to insertion through carrier 200, however, according to an alternate embodiment, tabs 47, 49 are bent outward after insertion through carrier 200. Dashed lines in Figure 6A illustrate yet another alternative embodiment of component E, wherein tabs 47, 49 extend both inward, toward one another, and outward. Figure 6B, as previously described, illustrates component E including centrally located tab 46 extending from electrode portion 413; tab 46 is shown including a projection 460 which extends toward a surface of second side 22 of carrier when tab 46 is bent, as illustrated in Figure 7B. Of course tab 46 need not include projection 460, and, according to alternate embodiments, tab 46 may be pre-formed, as shown in Figure 7B, prior to insertion through carrier 200. Those skilled in the art will recognize that various numbers and configurations of tabs, extending from electrode portion 413 of component E, may be employed within the scope of the present invention.

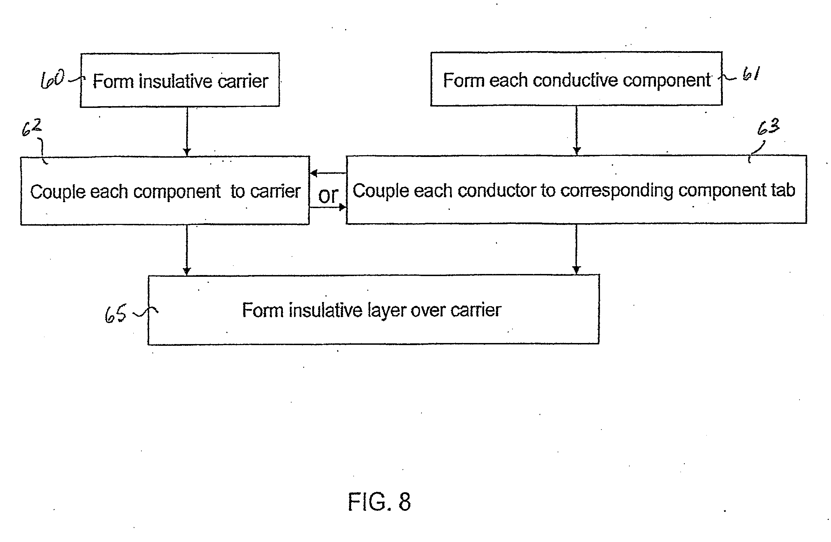

Figures 8 is a flow chart outlining alternate methods of the present invention for making inventive electrode assemblies. Figure 8 outlines methods wherein forming an insulative carrier, for example carrier 200, is one initial step 60 and forming each conductive component, for example, component E, is another initial step 61. After each component and the carrier is formed, a conductor is coupled to a tab of the corresponding component, per step 63, either before or after each component is coupled to the carrier, per step 62. In a latter step 65, an insulative layer is formed over the carrier to encapsulate each component tab and conductor. According to preferred methods, the carrier is formed (step 60) via a molding process, for example, transfer or injection molding, and a polymer mesh panel, for example, panel 25 (Figure 3D), is integrated into the carrier by inserting the panel into a mold prior to completing the molding process. A mold, which is used by some methods, includes features to form openings through the carrier and channels and recesses on first and second sides of the carrier, which openings, channels and recesses are described above in conjunction with carrier 200. According to some alternate methods, a process secondary to molding may be used to form channels and/or recesses, for example, by other types of thermal forming known to those skilled in the art, or by cutting or abrading methods, or by bonding additional layers to the molded carrier. For those embodiments of the carrier which include pre-formed openings, for example, openings 212, corresponding openings may be formed in the mesh panel prior to placing the panel in the mold, the panel openings being aligned with mold features for forming the openings of carrier, when the panel is placed.

Forming each conductive component (step 61) may be performed as described in conjunction with Figures 2A-B, and coupling each component to the carrier (step 62) is performed by inserting one or more tabs of the component through the carrier such that an electrode portion of the component is disposed on the first side of the carrier and the one or more tabs are disposed on the second side of the carrier to secure the component to the carrier, for example as illustrated in Figures 3A-B and 7A-B. The one or more tabs may be bent, after insertion through the carrier, or pre-formed in a bent configuration, prior to insertion through the carrier, such that bending the tabs after insertion through the carrier is not required to secure the component to the carrier. According to a preferred method of the present invention, the tabs of all of components E are bent or folded simultaneously after having been inserted through the carrier. All the tabs may be simultaneously folded by a plate that is pressed down toward the second side of the carrier while the components are supported, for example, along contact surfaces 43 of electrode portions 413.

According to some methods, after each component is coupled to the carrier (step 62), each conductor is coupled to the corresponding component tab, per step 63. According to some alternate methods, each conductor is coupled to the corresponding component tab (step 63) prior to coupling each component to the carrier (step 62). If step 62 follows step 63, a length of the conductor may be passed through the carrier ahead of the tab to couple the component to the carrier, or, the electrode portion of the component may be passed through the carrier, ahead of the tab. Although not shown in the outline of Figure 8, each conductor may be routed in grooves on the second side of the carrier either before or after coupling each conductor to the corresponding component tab. As previously described in conjunction with Figures 4B-D, Figure 5A, and method step 60, insulative carriers, according to some preferred embodiments of the present invention, include pre-formed channels which are suitable for routing conductors along the carrier to insulative tubular bodies and have features to hold conductors in place on the carrier while the insulative layer is formed over the carrier (step 65). Such channels may further provide some strain-relief for the coupling between each conductor and the corresponding component tab. Coupling each conductor to the corresponding component tab, per step 63, may be performed by any of the methods previously described, for example, by crimping, welding (e.g. laser or resistance), or a combination thereof, or by any other suitable method known to those skilled in the art.

Once each component and corresponding conductor, coupled thereto, are mounted or coupled to the carrier, an insulative layer may be formed, per step 65, over the side of the carrier on which the component tabs and conductors are disposed. As previously described, the insulative layer may further surround a portion of one or more elongate tubular bodies into which each conductor has been inserted, for example, portions AX, BX of tubular bodies A, B shown in Figures 5A-C. Each conductor may have been inserted into the corresponding tubular member at any point, before step 65, in the method outlined by Figure 8, that is, before or after any of steps 60, 61, 62, and 63. According to some methods of the invention, prior to insertion of each conductor into the corresponding tubular body, each tubular body is thermal formed to include a curved segment, for example segment 39, shown in Figure 5A, and may also be perforated to include an aperture, for example aperture 33, also shown in Figure 5A. Forming the insulative layer (step 65) is preferably performed by an over-molding process, for example, either by injection molding or transfer molding. According to the over-molding process, the carrier on which each electrode and corresponding conductor are mounted, along with each tubular body, in which each corresponding conductors has been inserted, are placed in a mold, and an insulative material, preferably silicone rubber, is injected into the mold to cover each conductor and component tab, for example, along second side 22 of carrier 200, and to surround each tubular body, for example, as illustrated in Figures 1A and 5A-C.

In the foregoing detailed description, the invention has been described with reference to specific embodiments. However, it may be appreciated that various modifications and changes can be made without departing from the scope of the invention as set forth in the appended claims.

## Claims

1. A method for making a medical electrical lead electrode assembly, the method comprising:
forming an insulative carrier (200) from an insulative material, the carrier including a first side (21) and a second side (22);
coupling at least one conductive component (E) to the carrier;
coupling an elongate flexible conductor (50, A1-8, B1-8) to the at least one tab of each of the at least one component;
inserting the conductor within an insulative tube (A, B);
forming a bend (39) in the tube prior to forming the insulative layer, the formed insulative layer extending about the bend in the tube;
forming an insulative layer (170) over the tab, the conductor and the insulative tube on the second side of the carrier, wherein the insulative layer couples the tube to the carrier; **characterized in that** the at least one conductive component (E) is coupled to the carrier by inserting at least one tab (42, 44, 46, 47, 49) of each of the at least one conductive component through the carrier from the first side to the second side and **in that** the at least one tab of each of the at least one conductive component extends away from an electrode portion (413) of the corresponding component such that, after each tab is inserted through the carrier, each tab is disposed on the second side of the carrier in order to secure the corresponding component to the carrier, and each electrode portion is disposed on the first side of the carrier with an inward facing surface (41) thereof disposed against the first side of the carrier.

2. The method of claim 1, wherein the at least one conductive component comprises a plurality of conductive components.

3. The method of claim 1, wherein forming the carrier comprises a molding process.

4. The method of claim 1, wherein the carrier further includes a pre-formed opening (212) corresponding to each tab, and inserting the at least one tab of each of the at least one component through the carrier comprises inserting each tab through the corresponding opening.

5. The method of claim 1, wherein coupling the conductor is performed prior to inserting the at least one tab.

6. The method of claim 1, wherein coupling the conductor to the tab of each of the at least one component comprises crimping.

7. The method of claim 1, wherein coupling the conductor to the tab of each of the at least one component comprises welding.

8. The method of claim 1, wherein the at least one tab comprises a pair of tabs and the conductor is coupled to one of the pair of tabs of each of the at least one component.

9. The method of claim 8, wherein the carrier further includes a pair of pre-formed openings corresponding to each pair of tabs and inserting the pair of tabs of each of the at least one conductive component through the carrier comprises inserting a first tab of each pair of tabs through a first opening of the pair of openings and inserting a second tab of the pair of tabs through a second opening of the corresponding pair of openings.

10. The method of claim 1, 2, 5, 6, 7 or 8, further comprising:
forming a slit in a mesh panel (25); and
placing the mesh panel in a mold;
wherein forming the carrier comprises molding the insulative material over the mesh panel in the mold, the mold including features for forming at least one opening in the carrier, the opening corresponding to the slit in the mesh panel and through which the at least one tab is inserted.

11. The method of claim 1, 2, 3, 4, 5, 6, 7, 8 or 9, further comprising disposing a portion of the conductor within a pre-formed channel (26, 28, BG1-8, AG1-8) of the carrier prior to forming the insulative layer.

12. The method of claim 11, wherein disposing the portion of the conductor is performed prior to coupling the conductor.

13. The method of claim 11, wherein disposing the portion of the conductor is performed after coupling the conductor.

14. The method of claim 1, 2, 3, 4, 5, 6, 7, 8 or 9, further comprising perforating a sidewall of the tube to form an aperture (33) therethrough, prior to forming the insulative layer, the insulative layer being formed to extend through the aperture.

15. The method of claim 1, 2, 3, 4, 5, 6, 7, 8 or 9, further comprising:
placing the carrier, the at least one conductive component coupled to the carrier, the conductor coupled to the tab of the at least one component, and the tube in a mold; and
wherein forming the insulative layer comprises molding the layer over the carrier, the at least one component, the conductor, and the tube in the mold.

## Patentansprüche

1. Verfahren zum Herstellen einer Elektrodenanordnung einer medizinischen elektrischen Leitung, wobei das Verfahren umfasst:
Formen eines isolierenden Trägers (200) aus einem Isoliermaterial, wobei der Träger eine erste Seite (21) und eine zweite Seite (22) aufweist;
Koppeln wenigstens einer leitenden Komponente (E) mit dem Träger;
Koppeln eines lang gestreckten, flexiblen Leiters (50, A1-8, B1-8) mit der wenigstens einen Kontaktfahne jeder der wenigstens einen Komponente;
Einsetzen des Leiters in ein isolierendes Rohr (A, B);
Bilden einer Biegung (39) in dem Rohr vor dem Formen der Isolierschicht, wobei die geformte Isolierschicht um die Biegung in dem Rohr verläuft;
Formen einer Isolierschicht (170) über der Kontaktfahne, dem Leiter und dem isolierenden Rohr auf der zweiten Seite des Trägers, wobei die Isolierschicht das Rohr mit dem Träger koppelt; **dadurch gekennzeichnet, dass** die wenigstens eine leitende Komponente (E) mit dem Träger durch Einsetzen wenigstens einer Kontaktfahne (42, 44, 46, 47, 49) jeder der wenigstens einen leitenden Komponente durch den Träger von der ersten Seite zu der zweiten Seite gekoppelt wird und dass sich die wenigstens eine Kontaktfahne jeder der wenigstens einen leitenden Komponente von einem Elektrodenabschnitt (413) der entsprechenden Komponente weg erstreckt, so dass, nachdem jede Kontaktfahne durch den Träger eingesetzt worden ist, jede Kontaktfahne auf der zweiten Seite des Trägers angeordnet ist, um die entsprechende Komponente an dem Träger zu befestigen, und jeder Elektrodenabschnitt auf der ersten Seite des Trägers angeordnet ist, wobei eine nach innen weisende Oberfläche (41) hiervon an der ersten Seite des Trägers angeordnet ist.

2. Verfahren nach Anspruch 1, wobei die wenigstens eine leitende Komponente mehrere leitende Komponenten enthält.

3. Verfahren nach Anspruch 1, wobei das Bilden des Trägers einen Gießprozess umfasst.

4. Verfahren nach Anspruch 1, wobei der Träger ferner eine im Voraus geformte Öffnung (212), die jeder Kontaktfahne entspricht, enthält und das Einsetzen der wenigstens einen Kontaktfahne jeder der wenigstens einen Komponente durch den Träger das Einsetzen jeder Kontaktfahne durch die entsprechende Öffnung umfasst.

5. Verfahren nach Anspruch 1, wobei das Koppeln des Leiters vor dem Einsetzen der wenigstens einen Kontaktfahne ausgeführt wird.

6. Verfahren nach Anspruch 1, wobei das Koppeln des Leiters mit der Kontaktfahne jeder der wenigstens einen Komponente ein Crimpen umfasst.

7. Verfahren nach Anspruch 1, wobei das Koppeln des Leiters mit der Kontaktfahne jeder der wenigstens einen Komponente ein Schweißen umfasst.

8. Verfahren nach Anspruch 1, wobei die wenigstens eine Kontaktfahne ein Paar Kontaktfahnen enthält und der Leiter mit einer des Paars von Kontaktfahnen jeder der wenigstens einen Komponente gekoppelt ist.

9. Verfahren nach Anspruch 8, wobei der Träger ferner ein Paar von im Voraus geformten Öffnungen aufweist, die jedem Paar von Kontaktfahnen entsprechen, und das Einsetzen des Paars von Kontaktfahnen jeder der wenigstens einen leitenden Komponente durch den Träger das Einsetzen einer ersten Kontaktfahne jedes Paars von Kontaktfahnen durch eine erste Öffnung des Paars von Öffnungen und das Einsetzen einer zweiten Kontaktfahne des Paars von Kontaktfahnen durch eine zweite Öffnung des entsprechenden Paars von Öffnungen umfasst.

10. Verfahren nach Anspruch 1, 2, 5, 6, 7 oder 8, das ferner umfasst:
Bilden eines Schlitzes in einer Gitterplatte (25); und
Anordnen der Gitterplatte in einer Gießform;
wobei das Formen des Trägers das Gießen des isolierenden Materials auf die Gitterplatte in der Gießform umfasst, wobei die Gießform Merkmale aufweist, um wenigstens eine Öffnung in dem Träger zu formen, wobei die Öffnung dem Schlitz in der Gitterplatte entspricht, durch die die wenigstens eine Kontaktfahne eingesetzt wird.

11. Verfahren nach Anspruch 1, 2, 3, 4, 5, 6, 7, 8 oder 9, das ferner das Anordnen eines Abschnitts des Leiters in einem im Voraus geformten Kanal (26, 28, BG1-8, AG1-8) des Trägers vor dem Formen der Isolierschicht umfasst.

12. Verfahren nach Anspruch 11, wobei das Anordnen des Abschnitts des Leiters vor dem Koppeln des Leiters ausgeführt wird.

13. Verfahren nach Anspruch 11, wobei das Anordnen des Abschnitts des Leiters nach dem Koppeln des Leiters ausgeführt wird.

14. Verfahren nach Anspruch 1, 2, 3, 4, 5, 6, 7, 8 oder 9, das ferner vor dem Formen der Isolierschicht das Durchlochen einer Seitenwand des Rohrs, um eine hindurchgehende Öffnung (33) auszubilden, umfasst, wobei die Isolierschicht so geformt ist, dass sie durch die Öffnung verläuft.

15. Verfahren nach Anspruch 1, 2, 3, 4, 5, 6, 7, 8 oder 9, das ferner umfasst:
Anordnen des Trägers, der wenigstens einen leitenden Komponente, die mit dem Träger gekoppelt ist, des Leiters, der mit der Kontaktfahne der wenigstens einen Komponente gekoppelt ist, und des Rohrs in einer Gießform; und
wobei das Formen der Isolierschicht das Gießen der Schicht auf den Träger, der wenigstens einen Komponente, dem Leiter und dem Rohr in der Gießform umfasst.

## Revendications

1. Procédé pour fabriquer un ensemble d'électrodes de fil électrique médical, le procédé comportant les étapes consistant à :
former un support isolant (200) à partir d'une matière isolante, le support incluant un premier côté (21) et un second côté (22) ;
coupler au moins un composant conducteur (E) au support ;
coupler un conducteur souple allongé (50, A1-8, B1-8) à la au moins une languette de chacun des au moins un composant ;
insérer le conducteur à l'intérieur d'un tube isolant (A, B) ;
former une courbure (39) dans le tube avant de former la couche isolante, la couche isolante formée s'étendant autour de la courbure dans le tube ;
former une couche isolante (170) au-dessus de la languette, du conducteur et du tube isolant sur le second côté du support, dans lequel la couche isolante couple le tube au support ;
**caractérisé en ce que** le au moins un composant conducteur (E) est couplé au support en insérant au moins une languette (42, 44, 46, 47, 49) de chacun des au moins un composant conducteur à travers le support du premier côté au second côté, et **en ce que** la au moins une languette de chacun des au moins un composant conducteur s'étend en s'éloignant d'une portion d'électrode (413) du composant correspondant de telle sorte que, après que chaque languette ait été insérée à travers le support, chaque languette est disposée sur le second côté du support afin de fixer le composant correspondant au support, et chaque portion d'électrode est disposée sur le premier côté du support avec une surface dirigée vers l'intérieur (41) de celui-ci disposée contre le premier côté du support.

2. Procédé selon la revendication 1, dans lequel le au moins un composant conducteur comporte une pluralité de composants conducteurs.

3. Procédé selon la revendication 1, dans lequel la formation du support comporte un processus de moulage.

4. Procédé selon la revendication 1, dans lequel le support inclut en outre une ouverture préformée (212) correspondant à chaque languette, et l'insertion de la au moins une languette de chacun des au moins un composant à travers le support comporte l'insertion de chaque languette à travers l'ouverture correspondante.

5. Procédé selon la revendication 1, dans lequel le couplage du conducteur est réalisé avant d'insérer la au moins une languette.

6. Procédé selon la revendication 1, dans lequel le couplage du conducteur à la languette de chacun des au moins un composant comporte un sertissage.

7. Procédé selon la revendication 1, dans lequel le couplage du conducteur à la languette de chacun des au moins un composant comporte un soudage.

8. Procédé selon la revendication 1, dans lequel la au moins une languette comporte une paire de languettes et le conducteur est couplé à une languette de la paire de languettes de chacun des au moins un composant.

9. Procédé selon la revendication 8, dans lequel le support inclut en outre une paire d'ouvertures préformées correspondant à chaque paire de languettes et l'insertion de la paire de languettes de chacun des au moins un composant conducteur à travers le support comporte l'insertion d'une première languette de chaque paire de languettes à travers une première ouverture de la paire d'ouvertures et l'insertion d'une seconde languette de la paire de languettes à travers une seconde ouverture de la paire correspondante d'ouvertures.

10. Procédé selon la revendication 1, 2, 5, 6, 7 ou 8, comportant en outre les étapes consistant à :
former une fente dans un panneau à mailles (25) ; et
placer le panneau à mailles dans un moule ;
dans lequel la formation du support comporte le moulage de la matière isolante au-dessus du panneau à mailles dans le moule, le moule incluant des caractéristiques pour former au moins une ouverture dans le support, l'ouverture correspondant à la fente dans le panneau à mailles et à travers laquelle la au moins une languette est insérée.

11. Procédé selon la revendication 1, 2, 3, 4, 5, 6, 7, 8 ou 9, comportant en outre la mise en place d'une portion du conducteur à l'intérieur d'un canal préformé (26, 28, BG1-8, AG1-8) du support avant de former la couche isolante.

12. Procédé selon la revendication 11, dans lequel la mise en place de la portion du conducteur est réalisée avant le couplage du conducteur.

13. Procédé selon la revendication 11, dans lequel la mise en place de la portion du conducteur est réalisée après le couplage du conducteur.

14. Procédé selon la revendication 1, 2, 3, 4, 5, 6, 7, 8 ou 9, comportant en outre la perforation d'une paroi latérale du tube pour former une ouverture (33) à travers celle-ci, avant de former la couche isolante, la couche isolante étant formée pour s'étendre à travers l'ouverture.

15. Procédé selon la revendication 1, 2, 3, 4, 5, 6, 7, 8 ou 9, comportant en outre l'étape consistant à :
placer le support, le au moins un composant conducteur couplé au support, le conducteur couplé à la languette des au moins un composant, et le tube dans un moule ; et
dans lequel la formation de la couche isolante comporte le moulage de la couche au-dessus du support, du au moins un composant, du conducteur et du tube dans le moule.
